# EUROPEAN PATENT APPLICATION

(11) **EP 2 008 583 A1**
(43) Date of publication of application: **31.12.2008**
(21) Application number: 08252222.8
(22) Date of filing: 27.06.2008
(51) Int. Cl.: A61B 5/03, A61M 27/00

(54) **Cradled sensory assembly**

(30) Priority: 29.06.2007 US 770998
(71) Applicant: Codman & Shurtleff, Inc., Raynham, Massachusetts 02767-0350 (US)
(72) Inventor: Dextradeur, Alex, Franklin, MA 02038 (US); Wilson, Sephen, North Easton, MA 02356 (US); McCusker, Daniel, Bridgewater, MA 02324 (US)
(74) Representative: Tunstall, Christopher Stephen

(57) **Abstract**

Various methods and devices are provided for removably coupling a sensor assembly to a catheter to measure conditions surrounding the catheter when the catheter is implanted in a patient. In one embodiment, a modular sensor assembly is provided and includes a housing having an antenna therein adapted to be removably coupled to at least a portion of a catheter.
The housing can be a closed loop having an opening therethrough. One or more sensors can be coupled to the antenna for measuring conditions surrounding the catheter when the catheter is implanted in a patient. In one embodiment, the housing can be adapted to removably couple to a distal end of the catheter, and the opening of the housing can be adapted to receive the catheter. The antenna can be a loop disposed within the housing.

## Description

### BACKGROUND

Hydrocephalus is a neurological condition that is caused by the abnormal accumulation of cerebrospinal fluid (CSF) within the ventricles, or cavities, of the brain. CSF is a clear, colorless fluid that is primarily produced by the choroid plexus and surrounds the brain and spinal cord. CSF constantly circulates through the ventricular system of the brain and is ultimately absorbed into the bloodstream. CSF aids in the protection of the brain and spinal cord. Because CSF keeps the brain and spinal cord buoyant, it acts as a protective cushion or "shock absorber" to prevent injuries to the central nervous system.

Hydrocephalus, which affects children and adults, arises when the normal drainage of CSF in the brain is blocked in some way. Such blockage can be caused by a number of factors, including, for example, genetic predisposition, intra-ventricular or intra-cranial hemorrhage, infections such as meningitis, head trauma, or the like. Blockage of the flow of CSF consequently creates an imbalance between the amount of CSF produced by the choroid plexus and the rate at which CSF is absorbed into the bloodstream, thereby increasing pressure on the brain, which causes the ventricles to enlarge.

Hydrocephalus can be treated by surgically inserting a reservoir or a shunt system that diverts the flow of CSF. Reservoirs (a Rickham reservoir is one well known type) are typically implanted outside the skull, and often under the skin, with a catheter that extends into the cranium for the purpose of drawing off CSF. A doctor can typically remove fluid from the reservoir using a hypodermic needle through the skin and reservoir cover in order to reduce pressure. Shunts work in a similar fashion, except rather than storing CSF as a reservoir does, shunts divert CSF from the ventricle to a drainage bag, or to another area of the body where the CSF can be absorbed as part of the circulatory system. Shunt systems come in a variety of models, and typically share similar functional components. These components include a ventricular catheter which is introduced through a burr hole in the skull and implanted in the patient's ventricle, a drainage catheter that carries the CSF to its ultimate drainage site, and optionally a flow-control mechanism, e.g., shunt valve, that regulates the one-way flow of CSF from the ventricle to the drainage site to maintain normal pressure within the ventricles.

One common problem encountered in the treatment of hydrocephalus is the difficulty in measuring the pressure of the patient's CSF. In one approach to this problem, a sensor is implanted for measuring pressure as in, for example, US 6,731,967 to Penn et al. While CSF pressure can be measured in this way, it necessarily involves a second implantation of a medical device when used in addition to a shunt or reservoir. A further technique involves implanting a shunt having a built in pressure sensor such that the sensor communicates with the cerebrospinal fluid, as in, for example, US 2006-0211944 to Mauge et al. While such devices can work well, combining such pressure sensing devices with a shunt results in an expensive device, resulting in the application of such combinations to only a few devices.

### SUMMARY

The present invention provided various methods and devices for removably coupling a sensor assembly to a catheter to measure conditions surrounding the catheter when the catheter is implanted in a patient. In one embodiment, the device can include a modular sensor assembly for use with a catheter. The sensor assembly can include a housing having an antenna therein adapted to be removably coupled to at least a portion of a catheter. The housing can be a closed loop having an opening therethrough. One or more sensors can be coupled to the antenna for measuring conditions surrounding the catheter when the catheter is implanted in a patient. In one embodiment, the housing can be adapted to removably couple to a distal end of the catheter, and the opening of the housing can be adapted to receive the catheter. The antenna can be a loop disposed within the housing.

The sensors of the sensor assembly can positioned in a variety of locations relative to the housing. In one embodiment, the housing can include a leg extending therefrom and along the catheter, with the leg having a proximal end coupled to a housing and distal end housing the one or more sensors. In another embodiment, the one or more sensors can be located within the housing. The sensors can be used to measure a variety of conditions surrounding the catheter. For example, the one or more sensors can include a pressure sensor for measuring intracranial pressure in the skull of the patient, or a temperature sensor for measuring temperature of the body of the patient.

The sensor assembly can also include a remote module adapted to communicate with the antenna in the housing to retrieve data measured by the one or more sensors. The remote module can be adapted to communicate with the antenna to couple power to the sensors. The remote module can communicate with the antenna in a variety of ways, e.g., wirelessly.

A modular system for measuring conditions within a patient is also provided, and can include a valve device for regulating fluid flow within a patient. The valve can have a catheter extending therefrom, with the catheter having a first end configured for attachment to the valve, a second end configured for fluid uptake, and a channel extending therebetween for carrying fluid within the catheter. The system also includes a sensor assembly removably coupled to the catheter for measuring conditions surrounding the valve device. The sensor assembly can include one or more sensors and a housing having an antenna therein. The housing can be a closed loop having an opening therethrough, with the opening being adapted to receive the catheter of the valve device therethrough. The sensor assembly can further include a remote module for reading measurements from the one or more sensors.

Methods for measuring conditions surrounding a catheter, such as sensing intracranial pressure, are also provided, and in one embodiment the method can include implanting a catheter under the skin of a user, with the catheter having a sensor assembly removably coupled thereto, measuring pressure surrounding the catheter using the sensor assembly, and reading the pressure measured by the sensor assembly using a remote module. In one embodiment, the sensor assembly can include one or more sensors and a housing having an antenna therein, with the one or more sensors being coupled to the antenna. The housing can be a closed loop having an opening therethrough which can be adapted to receive the catheter therethrough. The sensor can be positioned in a variety of locations relative to the housing. In one embodiment, the housing can have a leg extending therefrom, with the leg having a proximal end coupled to a housing and a distal end housing the one or more sensors. In another embodiment, the one or more sensors can be located within the housing. The remote module can communicate with the sensor assembly in a variety of ways, including using wireless communication.

### BRIEF DESCRIPTION OF THE DRAWINGS

The invention will be more fully understood from the following detailed description taken in conjunction with the accompanying drawings, in which:

FIG. 1A is a side view of one embodiment of a sensor assembly having a housing with an antenna disposed therein and a leg extending from the housing and having a sensor disposed in a distal end thereof;

FIG. 1B is a perspective view of the sensor assembly shown in FIG. 1A;

FIG. 2 is a side view of another embodiment of a sensor assembly having a housing with an antenna and a sensor disposed therein;

FIG. 3 is a side view of the sensor assembly shown in FIG. 1A-1B removably coupled to a valve and catheter assembly;

FIG. 4 is a cross-sectional view of a further embodiment of a sensor and valve assembly; and

FIG. 5 is a perspective view of a sensor assembly implanted within a patient, and a remote module communicating with an antenna of the sensor assembly.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for a sensor assembly that can be removably coupled to a reservoir or shunt system. The sensor assembly can have one or more sensors and an antenna that is coupled to the sensors. The sensors and the antenna can be coupled in a variety of ways, including by a connector. The sensors can be adapted to measure conditions in the patient surrounding the sensors, and the antenna can be adapted to communicate the measurements to a remote module.

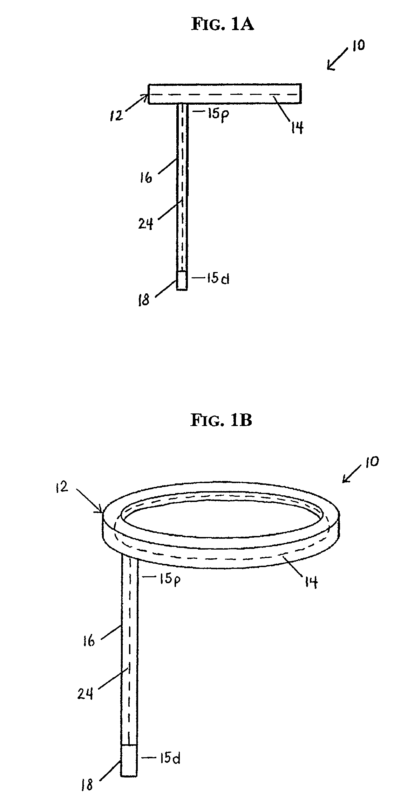

FIGS. 1A-1B illustrates one exemplary embodiment of a sensor assembly for use with a catheter to measure conditions surrounding the catheter when the catheter is implanted in a patient. As shown in FIGS. 1A-2, a sensor assembly 10 generally includes a housing 12 having an antenna 14 (illustrated as ghosted within the housing 12 in the FIGS.) disposed therein and a sensor 18 coupled to the antenna 14.

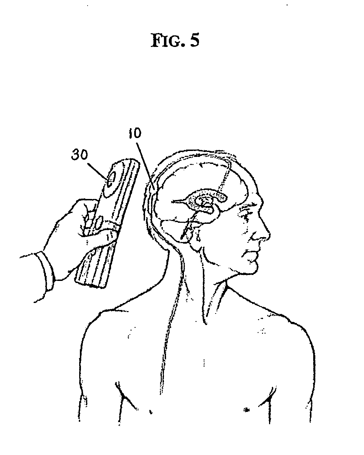

The housing 12 and the antenna 14 can be any shape and size that is capable of removably coupling to the catheter, but in one embodiment, both the housing 12 and the antenna 14 can be a closed loop having an opening therethrough for receiving the catheter, as will be discussed in more detail below. The housing 12 can be formed from any biocompatible material suitable for implanted medical devices, including stainless steel and medical plastics. The antenna 14 can also have a variety of configurations, but in an exemplary embodiment, the antenna 14 has a coil-shaped configuration. In particular, as illustrated in FIGS 1A-2, the antenna 14 is formed into a substantially circular coil. The coil configuration will allow the antenna 14 to communicate with an external device, such as the radiofrequency telemetry device 30 illustrated in FIG. 5.

The sensor 18 can be positioned in a variety of locations relative to the housing 12. In one exemplary embodiment shown in FIGS. 1A-1B, the housing 12 can include a leg 16 extending therefrom, and the leg 16 can have a proximal end 15p coupled to the housing 12 and a distal end 15d that can house the sensor 18. The leg 16 can be any size and shape that can facilitate placement of the sensor 18, but is generally an elongate cylinder having the sensor 18 disposed within the distal end 15d thereof. The leg 16 can have a lumen therethrough and extending along its length to allow a connector 24 to couple the sensor 18 to the antenna 16 located within the housing 12. The leg 16 can be of any length required to place the sensor 18 in a location at which the condition surrounding the sensor 18 will be measured. The leg 16 can extend from the housing 12 in such a way as to extend along a portion of the length of the catheter.

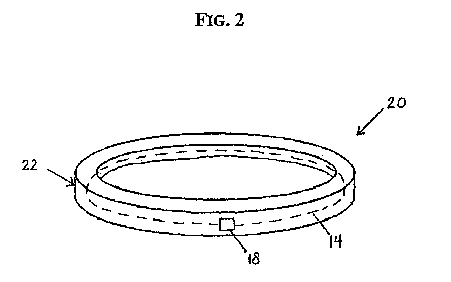

The leg 16 can be can be flexible or rigid depending on the placement of the leg 16 within the patient. For example, the leg 16 can be flexible and adapted to be inserted into a lumen of the body, including a ventricle in the brain of the patient along side the catheter of a shunt. The leg 16 can also be rigid and adapted to be inserted into dense body tissue, such as the parenchyma of the brain. The leg 16 can be formed from a variety of materials. In an exemplary embodiment, however, the leg 16 is formed from a flexible biocompatible material. Suitable materials include, for example, polymers such as silicones, polyethylene, and polyurethane. In addition, the leg 16 may be formed of, or include, a radio-opaque material so that its placement can be verified using non-invasive imaging equipment. A person skilled in the art will appreciate that the leg 16 which houses the sensor 18 can be inserted into any location in the body at which a measurement of the surrounding conditions needs to be taken. In another exemplary embodiment of a sensor assembly 20 shown in FIG. 2, the sensor can be disposed within a 22 housing of the sensor assembly 20. Still further, the sensor 18 itself can be encapsulated for implantation separately while being tethered to housing 12 and antenna 14 using connector 24. A person skilled in the art will appreciate that the sensor can be housed in a variety of ways and locations relative to the housing of the sensor assembly so long as the sensor can be coupled to the antenna within the housing.

Virtually any type of sensor can be used with the sensor assembly to measure a variety of different conditions in the environment surrounding the catheter. In one embodiment, the sensor can be a pressure sensor that is adapted to measure a pressure of an external environment, e.g., the pressure of CSF in the ventricle of the patient's brain, surrounding the catheter. Exemplary pressure sensing chips (as well as antennas for communicating with the chips) are illustrated and described in US 5,321,989 to Zimmer et al.; US 5,431,057 to Zimmer et al.; and EP 1 312 302 to Boedecker et al.; each of which is expressly incorporated by reference herein. In a further embodiment, the sensor can also include temperature sensor that is adapted to measure the temperature of the environment in which the pressure measurement is made. A person skilled in the art will appreciate that the sensor of the sensor assembly can be any type of sensor and that the sensor assembly can house any number of sensors to measure a variety of different conditions in a patient.

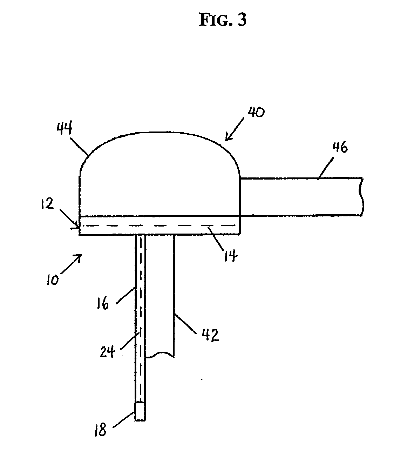

The sensor assembly can be removably coupled to a reservoir or shunt system (generally, valve system 40) in a variety of ways. In one exemplary embodiment, the sensor assembly 10 can be coupled to the valve system 40 before the valve system 40 is implanted in a patient, as shown in FIG. 3. In general, valve system 40 is a right-angle, or Rickham-type, hydrocephalus valve. It includes a catheter 42 for implantation into a patient's ventricle for drawing cerebrospinal fluid into a reservoir 44. The reservoir 44 may further include an outlet lumen 46, which can further be connected to an in-line valve (not shown). The sensor housing 12 can cradle the base of reservoir 44 using existing features of the Rickham valve for implantation on top of the skull. Sensor 18 may be implanted through the same cranial access hole used by the valve system 40, or may use a separate access hole. While this can add slightly to the height of the combined sensor-valve device, implantation is straightforward and sensor functionality can be added to many common off-the-shelf hydrocephalus valve systems in this way.

In another exemplary embodiment, the sensor assembly 10 can be implanted in the patient prior to implantation of the catheter. The catheter can then be coupled to the sensor assembly 10 as the catheter is implanted by inserting the catheter through the opening of the housing 12 of the sensor assembly until the housing 12 is located just proximal of the distal end of the catheter, allowing the catheter and the sensor assembly 10 to removably couple to one another.

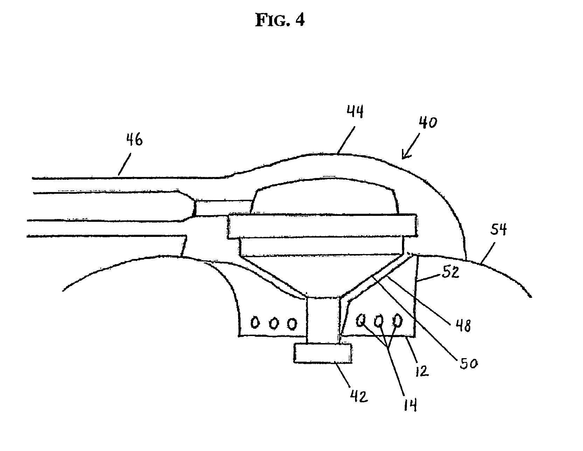

A further embodiment is illustrated in FIG. 4. In this embodiment, housing 12 is implanted into a burr-hole 52 in the patient's bone 54. The right-angle valve 40 includes a base 50, as well as reservoir 44, outlet lumen 46, and catheter input 42. The housing 12 includes a beveled superior surface that corresponds to an inferior surface on the base 50, and a central opening in the housing 12 corresponds to the catheter input 42 on the base 50. In this way, a burr-hole 52 can be sized and shaped to hold housing 12, and the valve assembly 40 can be mated directly to the housing to form a complete sensing and valve system.

In one embodiment, the antenna can be adapted to communicate conditions sensed by the sensor to an external remote module. The remote module can receive information from the sensor assembly by a variety of techniques, including wired or wireless communication. The communication between the remote module and the antenna is preferably wireless, as shown in FIG. 5. This allows the sensor assembly 10 to be completely implanted within the patient with no external components extending from the patient to allow a connection to a remote module 30. The remote module 30 can be positioned near the antenna 14 in the housing 12 to retrieve information obtained from the sensor 18 of the sensor assembly 10, and in addition, to couple power to the sensor 18. The range at which the remote module 30 can be positioned can vary, but in one exemplary embodiment, the remote module 30 can be positioned between 20-50 mm from the antenna for the remote module 30 to be able to communicate wirelessly with the antenna 14.

In one embodiment, the sensor assemblies described above can be used with a shunt system and implanted in the brain of a patient. In use, the catheter of the shunt system can be positioned in the patient's ventricle, and the sensor assembly and the valve of the shunt system can be positioned just beneath the patient's scalp. The sensor, e.g., a pressure sensor, which extends from the housing of the sensor assembly and into the area surrounding the catheter, can measure the ventricular pressure surrounding the catheter in the brain of the patient. The remote module can then be positioned adjacent to the antenna disposed within the housing to communicate with the antenna, and thereby obtain a reading of the measured pressure within the ventricle of the patient. A person skilled in the art will appreciate that, while the sensor assembly is described in connection with being removably coupled to a catheter of a shunt system disposed in the ventricle, the sensor assembly can be coupled to any valve and catheter system and disposed at a variety of other locations within the body and in a variety of other devices.

One of ordinary skill in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A modular sensor assembly for use with a catheter, comprising:
a housing having an antenna therein adapted to be removably coupled to at least a portion of a catheter, the housing being a closed loop having an opening therethrough; and
one or more sensors coupled to the antenna for measuring conditions surrounding the catheter when the catheter is implanted in a patient.

2. The assembly of claim 1, wherein the housing is adapted to removably couple to a distal end of the catheter.

3. The assembly of claim 2, wherein the opening of the housing is adapted to receive the catheter.

4. The assembly of claim 1, wherein the antenna is a loop disposed within the housing.

5. The assembly of claim 1, wherein the housing includes a leg extending therefrom and along the catheter, the leg having a proximal end coupled to a housing and distal end housing the one or more sensors.

6. The assembly of claim 1, wherein the one or more sensors include a pressure sensor for measuring intracranial pressure in the skull of the patient or a temperature sensor for measuring temperature of the body of the patient.

7. The assembly of claim 1, further including a remote module adapted to communicate with the antenna in the housing to retrieve data measured by the one or more sensors.

8. The assembly of claim 7, wherein the remote module is adapted to communicate with the antenna to couple power to the sensor.

9. The assembly of claim 7, wherein the remote module communicates with the antenna wirelessly.

10. A modular system for measuring conditions within a patient, comprising:
a valve device for regulating fluid flow within a patient, the valve having a catheter extending therefrom, the catheter having a first end configured for attachment to the valve, a second end configured for fluid uptake, and a channel extending therebetween for carrying fluid within the catheter; and
a sensor assembly removably coupled to the catheter for measuring conditions surrounding the valve device.

11. The system of claim 10, wherein the sensor assembly includes one or more sensors and a housing having an antenna therein.

12. The system of claim 11, wherein the sensor assembly further includes a remote module for reading measurements from the one or more sensors.

13. A method for sensing intracranial pressure, comprising:
implanting a valve assembly under the skin of a user, the valve assembly including a catheter and having a sensor assembly removably coupled thereto;
measuring pressure surrounding the catheter using the sensor assembly; and
reading the pressure measured by the sensor assembly using a remote module.

14. The method of claim 13, wherein the sensor assembly includes one or more sensors and a housing having an antenna therein, the one or more sensors being coupled to the antenna.

15. The system of claim 11 or the method of claim 14, wherein the housing is a closed loop having an opening therethrough, the opening being adapted to receive the catheter therethrough.

16. The method of claim 14, wherein the housing has a leg extending therefrom, the leg having a proximal end coupled to a housing and a distal end housing the one or more sensors.

17. The assembly of claim 1 or the method of claim 14, wherein the one or more sensors are located within the housing.

18. The method of claim 13, wherein the remote module communicates with the sensor assembly using wireless communication.
